# FASCICULE DE BREVET EUROPEEN

(11) **EP 3 458 117 B1**
(45) Date de publication et mention de la délivrance du brevet: **04.11.2020**
(21) Numéro de dépôt: 17731215.4
(22) Date de dépôt: 19.05.2017
(51) Int. Cl.: A61L 27/10, A61L 27/20, A61L 27/46, A61L 27/58

(54) **PRODUIT ET PROCEDE POUR LA PREPARATION D'UNE COMPOSITION INJECTABLE, A BASE DE CHITOSANE ET DE BIOVERRES, DESTINEE A LA REGENERATION DU TISSU OSSEUX**
PRODUKT UND VERFAHREN ZUR HERSTELLUNG EINER INJIZIERBAREN ZUSAMMENSETZUNG AUF BASIS VON CHITOSAN UND BIOGLÄSERN ZUR REGENERATION VON KNOCHENGEWEBE
PRODUCT AND PROCESS FOR THE PREPARATION OF AN INJECTABLE COMPOSITION BASED ON CHITOSAN AND BIOGLASSES, INTENDED FOR THE REGENERATION OF BONE TISSUE

(30) Priorité: 19.05.2016 FR 1654470
(43) Date de publication de la demande: 27.03.2019
(73) Titulaire: Université Claude Bernard Lyon I, 69100 Villeurbanne (FR); Institut National des Sciences Appliquées de Lyon, 69621 Villeurbanne Cedex (FR); Université Jean Monnet Saint Etienne, 42100 Saint Etienne (FR); Centre National de la Recherche Scientifique, 75016 Paris (FR); Université de Montpellier, 34090 Montpellier (FR)
(72) Inventeur: MONTEMBAULT, Alexandra, 42800 RIVE DE GIER (FR); DAVID, Laurent, 69004 Lyon (FR); DELAIR, Thierry, 69700 Echalas (FR); TADIER, Solène, 69004 Lyon (FR); GREMILLARD, Laurent, 42100 Saint-Etienne (FR); FAIVRE, Annelise, 34730 Prades Le Lez (FR); DESPETIS, Florence, Prades Le Lez 34730 (FR)
(74) Mandataire: Be IP
(86) Numéro de dépôt international: PCT/FR2017/051231
(87) Numéro de publication internationale: WO 2017/198970

(56) Documents cités:
- DATABASE WPI Week 201033 Thomson Scientific, London, GB; AN 2010-E77899 XP002766581, & CN 101 695 584 A (UNIV ZHEJIANG) 21 avril 2010 (2010-04-21)
- SAMIRA JEBAHI: "Repair of bone defect using bioglass-chitosan as a pharmaceutical drug: An experimental study in an ovariectomised rat model", AFRICAN JOURNAL OF PHARMACY AND PHARMACOLOGY, vol. 6, no. 16, 29 avril 2012 (2012-04-29) , XP055340705, DOI: 10.5897/AJPP12.214
- COUTO D S ET AL: "Development of bioactive and biodegradable chitosan-based injectable systems containing bioactive glass nanoparticles", ACTA BIOMATERIALIA, ELSEVIER, AMSTERDAM, NL, vol. 5, no. 1, 1 janvier 2009 (2009-01-01) , pages 115-123, XP025804527, ISSN: 1742-7061, DOI: 10.1016/J.ACTBIO.2008.08.006 [extrait le 2008-08-26]
- GISELA M LUZ ET AL: "Chitosan/bioactive glass nanoparticles composites for biomedical applications", BIOMEDICAL MATERIALS, vol. 7, no. 5, 12 septembre 2012 (2012-09-12), page 054104, XP055340715, GB ISSN: 1748-6041, DOI: 10.1088/1748-6041/7/5/054104

## Description

La présente invention vise à proposer un produit bi-composant dédié à la préparation d'une formulation injectable, dont l'application concerne la régénération du tissu osseux après injection dans l'organisme d'un sujet. Plus précisément, les domaines d'application envisagés sont le traitement des pertes de substances osseuses en orthopédie ou en odontologie.

Les verres bioactifs, également nommés bioverres, sont connus en tant que matériaux céramiques ou de type verres, qui sont aptes à développer à leur surface, en présence d'un milieu aqueux, ou à leur interface avec un tissu corporel, une couche d'hydroxyapatite phosphocalcique carbonatée cristallisée, produisant ainsi une réponse utile biologiquement. Cette couche d'hydroxyapatite est, par sa composition, similaire à la phase minérale de l'os.

La notion de verres bioactifs inclut à la fois les verres au sens strict du terme et les matériaux vitrocéramiques, partiellement cristallisés, c'est-à-dire contenant à la fois au moins une phase amorphe formée de verre bioactif et au moins une phase cristalline, la phase amorphe n'étant pas nécessairement majoritaire. Pour plus de détails sur les verres bioactifs, on pourra se référer à l'ouvrage « BIOMATERIALS SCIENCE, An Introduction to Materials in Medicine, 2d Edition by Buddy D. Ratner, Allan S. Hoffman, Frederick J. Schoen, Jack E. Lemons, Elsevier Academic Press, chapter 2.10 Ceramics, Glasses, and Glass-Ceramics 153 Larry L. HENCH and Serena BEST » (2004), ainsi qu'à l'ouvrage « Bioactive glasses, Materials, properties and applications, Edited by Heimo O. Ylänen, Woodhead Publishing » (2011).

Les verres bioactifs peuvent être préparés de différentes façons, notamment comme décrit dans les techniques de l'Ingénieur (n4955 « Verres bioactifs », publiées le 10 avril 2014). Le pouvoir bioactif peut être testé selon la norme ISO 23317.

Dans l'art antérieur, des tentatives d'associer les verres bioactifs au chitosane, polysaccharide connu pour être biocompatible, biorésorbable, bactériostatique et fongistatique, ont été proposées. Oudadesse et al. (Jebahi S, Saoudi M, Farhat L, Oudadesse H, Rebai T, Kabir A, El Feki A, Keskes H. Effect of novel curcumin-encapsulated chitosan-bioglass drug on bone and skin repair after gamma radiation: experimental study on a Wistar rat model, Cell Biochem Funct, 2015, 33, 150-159) ont travaillé sur l'élaboration de matériaux composite bioverres/chitosane pour des applications en reconstruction osseuse et pour la cicatrisation des plaies dermiques. Les auteurs proposent de mélanger une solution de chitosane préparée en milieu acide et une suspension de bioverres en milieu acide, et ensuite, d'augmenter le pH du mélange obtenu par ajout de soude. L'ajout de soude induit la gélification du mélange qui est ensuite lavé et lyophilisé.

D'autres solutions de l'art antérieur proposent d'utiliser un mélange chitosane/bioverre contenant du glycérophosphate, qui, à 37°C, forme un gel avec la solution de chitosane contenant le bioverre. On peut notamment citer D. S. Couto, Z. Hong, J. F. Mano Development of bioactive and biodegradable chitosan-based injectable systems containing bioactive glass nanoparticles. Acta Biomaterialia 5 (2009) 115-123.Le document CN 101 695 584 A décrit une composition injectable comprenant une solution aqueuse de chitosane et des particules d'un ou plusieurs verres bioactifs. Cette composition comprend en plus un polymère d'acide hyaluronique. Ce document ne décrit pas le pH de la solution de chitosane. De plus, ni la masse moléculaire du chitosane ni le degré d'acétylation dudit chitosane n'y sont divulgués.

Ces différentes techniques ne donnent pas satisfaction, car, soit elles ne conduisent pas à des solutions injectables, soit elles utilisent des agents de réticulation posant des problèmes de toxicité.

Dans ce contexte, la présente invention vise à proposer une solution répondant à ces problèmes et concerne un produit destiné à la préparation d'une composition injectable. Le produit selon l'invention se doit de permettre une préparation simple et rapide d'une composition injectable adaptée à la régénération des tissus osseux.

L'invention concerne un produit, destiné à la préparation d'une composition injectable, contenant de manière séparée l'une de l'autre, au moins une première et une deuxième compositions, et dans lequel :
- la première composition dite A est une solution aqueuse de chitosane de pH appartenant à la gamme allant de 3 à 7, le chitosane présentant une masse moléculaire moyenne Mw appartenant à la gamme allant de 80 à 1000 kg/mol et un degré d'acétylation appartenant à la gamme allant de 0 à 60%, et
- la deuxième composition dite B1 correspond à des particules d'un ou plusieurs verre(s) bioactif(s),
   ledit produit ne contenant pas de glycérophosphate.

Le produit selon l'invention comprend également une composition C correspondant à de l'eau ou à une solution aqueuse (en particulier, du type solution tampon ou milieu de culture) de pH appartenant à la gamme allant 5,5 à 8, de préférence à la gamme allant de 6,8 à 7,2. Dans ce cas, la composition C sera, de préférence, destinée à être mélangée avec la composition B1, pour former une composition intermédiaire B2 correspondant à une suspension aqueuse de particules d'au moins un verre bioactif, le pH de cette suspension étant dans la gamme allant de 5,5 à 8. La masse de la composition B1 ou des compositions A, B1 et C respectivement, appartient à la gamme allant de 20% à 90%.

De manière avantageuse, le produit selon l'invention est exclusivement constitué des compositions A et B1, voire des compositions A, B1 et C.

De manière préférée, le produit selon l'invention ne contient pas de génépine, ni de glutaraldéhyde, ni de formaldéhyde. Plus généralement, le produit selon l'invention ne contient pas d'agent de réticulation.

Lorsque l'on dit que le produit selon l'invention ne contient pas un composant, cela signifie que ledit composant n'est présent ni dans la composition A, ni dans la composition B1, ni dans une autre composition C que le produit pourrait contenir.

Dans le cadre de l'invention, le mélange des compositions A et B1, ou des compositions A et B2, voire des compositions A, B1 et C, donne une composition injectable, qui va ensuite conduire à la formation d'un gel physique de chitosane. Aucun agent de réticulation conduisant à un gel chimique n'est utilisé. La solution selon l'invention est donc moins toxique que les solutions antérieures utilisant de tels agents de réticulation, puisqu'aucun agent de réticulation qui resterait ensuite dans l'organisme n'est présent.

Dans le cadre de l'invention, l'approche est totalement différente des solutions antérieures utilisant du glycérophosphate, puisque ce sont les particules de verre bioactif qui sont utilisées pour obtenir, à court terme, la gélification du chitosane. Le produit selon l'invention conduit, après mélange des compositions A et B1, ou des compositions A et B2, voire des compositions A, B1 et C, à une composition injectable pouvant être utilisée pour la régénération des tissus osseux, et donc, en orthopédie et en odontologie. Une telle composition injectable correspond à une suspension de particules d'au moins un verre bioactif dans une solution aqueuse de chitosane. Le mélange des compositions A et B1, ou des compositions A et B2, voire des compositions A, B1 et C, conduit, tout d'abord, à une composition injectable, qui va évoluer au cours du temps pour conduire à une gélification du chitosane, du fait d'un échange de cations entre les particules de verre(s) bioactif(s) et le chitosane. Dans le cadre de l'invention, il est donc possible d'obtenir une gélification qui intervient *in situ,* et donc *in vivo,* après injection de la composition dans l'organisme, ce qui permet le maintien des particules de verre bioactif sur le site d'implantation.

La composition injectable de verre(s) bioactif(s) obtenue présente une tolérance améliorée par rapport à une simple suspension de verre(s) bioactif(s), du fait de la présence du chitosane. En effet, les verres bioactifs classiques, lorsqu'ils sont utilisés seuls, entraînent une augmentation importante du pH au niveau du site d'implantation, et s'accompagnent d'une certaine toxicité, voire d'une nécrose tissulaire. La présence de la solution aqueuse de chitosane, au pH sélectionné, va permettre de contrôler l'augmentation de pH obtenu, en particulier, au sein de l'organisme. De même, la solution de chitosane, utilisée seule, présenterait une certaine toxicité, vis-à-vis de l'organisme, du fait de son caractère acide. L'utilisation combinée des deux permet d'atteindre dans l'organisme des pH dans la gamme allant de 6,5 à 7,5, notamment de 6,8 à 7,4, en fonction du pH initial de la solution de chitosane notamment, ce qui est bien adapté aux implants injectables.

L'état initial (c'est-à-dire peu de temps après mélange des compositions A et B1, ou des compositions A et B2, voire des compositions A, B1 et C) de la composition injectable, combinant le chitosane et les verres bioactifs, permet son injection et la formation *in situ* de l'hydrogel composite qui en résulte après injection, permet le maintien du matériau composite sur le site d'implantation.

La présence des particules de verre(s) bioactif(s) dans la composition injectable obtenue après mélange des compositions A et B1 ou des compositions A et B2, voire des compositions A, B1 et C, permet une immobilisation au niveau du tissu osseux. La présence du chitosane apporte des propriétés angiogéniques, stimule la reconstruction tissulaire et permet de combler de plus grands défauts osseux qu'avec l'utilisation de verres bioactifs seuls. L'association des deux compositions A et B1 ou des compositions A et B2, voire des compositions A, B1 et C, permet de pouvoir exploiter à la fois les propriétés rhéologiques et biologiques du chitosane et les propriétés bioactives des particules du ou des bioverre(s) sélectionné(s).

De par le choix de certains paramètres, que sont en particulier le pH de la composition A, la quantité de verre bioactif, les tailles des particules de bioverre, il est possible de contrôler le temps séparant le moment du mélange et celui de la gélification.

L'invention a également pour objet les compositions injectables, exemptes de glycérophosphate et contenant des particules d'un ou plusieurs verre(s) bioactif(s) en suspension dans une solution aqueuse de chitosane, obtenues par mélange des compositions A et B1, ou des compositions A et B2, voire des compositions A, B1 et C, du produit défini dans la présente description. De telles compositions sont qualifiées d'injectables, car elles sont dans un état antérieur à l'étape de gélification qui a vocation à intervenir *in situ,* en particulier, après injection de la composition dans l'organisme du sujet à traiter. En particulier, de telles compositions injectables présentent une viscosité pour une vitesse de cisaillement de 10⁴ s⁻¹ dans la gamme allant de 0,005 à 200 Pa.s. Cette viscosité pourra être mesurée par rhéométrie capillaire, notamment à 22°C, et en particulier selon la méthode décrite dans la publication « Chitosan solutions as injectable systems for dermal filler applications: rheological characterization and biological evidence » par C. Halimi, A. Montembault, A. Guerry, T. Delair, E. Viguier, R. Fulchiron, L. David. Annual International Conférence of the IEEE Engineering in Medicine and Biology Society. IEEE Engineering in Medicine and Biology Society. 2015; 2596-9. Les compositions injectables selon l'invention présentent avantageusement un pH dans la gamme allant de 3,1 à 7,4, de préférence, dans la gamme allant de 5 à 7. Les caractéristiques de viscosité et de pH données pour la composition injectable correspondent, de préférence, à celles obtenues immédiatement après le mélange des différents constituants utilisés pour leur préparation, et en particulier, des compositions A et B1, ou des compositions A et B2, voire des compositions A, B1 et C, en fonction du mode de mélange sélectionné, et notamment moins de 5 secondes après ledit mélange.

De manière avantageuse, les compositions injectables sont obtenues exclusivement à partir des compositions A et B1, ou bien des compositions A et B2, ou bien des compositions A, B1 et C. En particulier, il n'est pas nécessaire d'ajouter une base, telle que la soude pour obtenir une gélification. La gélification du chitosane présent dans la composition A est obtenue grâce à un verre bioactif présent dans la composition B1. Une telle composition injectable est donc, de préférence, obtenue par mélange des seules compositions A et B1 ou bien des seules compositions A et B2, ou bien des seules compositions A, B1 et C. La composition B1 correspond à des particules d'un verre bioactif ou à un mélange de particules de plusieurs verres bioactifs et se trouve donc sous la forme d'une poudre. Il est possible de mettre préalablement au mélange avec la composition A, cette poudre en suspension dans une solution aqueuse, et en particulier, dans de l'eau correspondant à la composition C. Dans ce cas, cela revient à former intermédiairement une suspension aqueuse B2 de particules d'au moins un verre bioactif, le pH de cette suspension étant, de préférence, dans la gamme allant de 5,5 à 8, de préférence dans la gamme allant de 6,8 à 7,2.

L'invention a également pour objet les produits destinés à la préparation d'une composition injectable et lesdites compositions injectables, décrits dans le cadre de l'invention, pour leur utilisation dans la régénération d'un tissu osseux, après injection de la composition injectable à un sujet, notamment dans le domaine orthopédique ou orthodontique. Le sujet sera un être humain ou un animal. En particulier, dans une telle utilisation après injection à un sujet, il y a formation d'un hydrogel de chitosane *in vivo.* La gélification du chitosane présent initialement dans la composition A est obtenue grâce à un verre bioactif présent initialement dans la composition B1.

L'invention concerne également une méthode de régénération d'un tissu osseux chez un sujet comprenant :
- le mélange des compositions A et B1, ou bien des compositions A et B2, ou bien des compositions A, B1 et C, conduisant à la formation d'une composition injectable, exempte de glycérophosphate contenant des particules d'un ou plusieurs verre(s) bioactif(s) en suspension dans une solution aqueuse de chitosane, en particulier selon le procédé de préparation décrit ci-après,
- l'injection audit sujet de la composition injectable obtenue au niveau de la zone où la régénération du tissu osseux est souhaitée,
- la gélification *in situ* de la composition injectée.

L'invention concerne également une méthode de régénération d'un tissu osseux chez un sujet comprenant :
- le mélange d'une solution aqueuse de chitosane de pH appartenant à la gamme allant de 3 à 7, dite composition A et d'une poudre de particules d'un ou plusieurs verre(s) bioactif(s), dite composition B1, en l'absence de glycérophosphate, conduisant à la formation d'une composition injectable, exempte de glycérophosphate, contenant des particules d'un ou plusieurs verre(s) bioactif(s) en suspension dans une solution aqueuse de chitosane, en particulier selon le procédé de préparation décrit ci-après,
- l'injection audit sujet de la composition injectable obtenue au niveau de la zone où la régénération du tissu osseux est souhaitée,
- la gélification *in situ* de la composition injectée.

L'invention concerne également une méthode de régénération d'un tissu osseux chez un sujet comprenant :
i) la préparation d'une composition injectable, exempte de glycérophosphate, contenant des particules d'un ou plusieurs verre(s) bioactif(s) en suspension dans une solution aqueuse de chitosane, caractérisé en ce qu'il comprend, voire consiste exclusivement en les étapes successives suivantes :
   - disposer d'une solution aqueuse de chitosane de pH appartenant à la gamme allant de 3 à 7, dite composition A,
   - d'une poudre de particules d'un ou plusieurs verre(s) bioactif(s), dite composition B1,
   - mettre la composition B1 en suspension aqueuse, et préférentiellement, en suspension dans de l'eau,
   - mélanger la composition A et la suspension aqueuse de particules d'au moins un verre bioactif obtenue, rapidement après la mise en suspension de la composition B1, de préférence, moins de 20 secondes après, et préférentiellement, moins de 10 secondes après,
   les différentes compositions et solutions utilisées étant exemptes de glycérophosphate,
ii) l'injection audit sujet de la composition injectable obtenue au niveau de la zone où la régénération du tissu osseux est souhaitée,
iii) la gélification *in situ* de la composition injectée.

L'invention concerne également un dispositif de conditionnement comprenant deux compositions A et B1 formant un produit selon l'invention, voire trois compositions A, B1 et C. Ledit dispositif peut être composé d'un unique ensemble de conditionnement comprenant les compositions A et B1 conditionnées séparément l'une de l'autre dans deux compartiments dudit ensemble de conditionnement, et en particulier, dans un système d'injection tel qu'une seringue à deux compartiments.

Selon un mode de réalisation particulier, le dispositif de conditionnement selon l'invention comprendra également la composition C, ledit dispositif pourra alors être composé d'un unique ensemble de conditionnement comprenant au moins trois compartiments indépendants contenant respectivement chacune des trois compositions A, B1 et C. Le dispositif comprendra, de préférence, des moyens pour permettre la mise en contact et le mélange des deux compositions A et B1, voire des trois compositions A, B1 et C. En particulier, les moyens permettant la mise en contact des deux compositions, voire des trois compositions, sont tels que le mélange desdites compositions est effectué au sein dudit ensemble de conditionnement juste avant l'injection de la composition injectable obtenue. Ces moyens pourront permettre d'effectuer un mélange séquentiel, lorsque le dispositif contient les compositions A, B1 et C : mise en contact et mélange des compositions B1 et C, pour former une composition intermédiaire B2, puis, mise en contact et mélange des compositions B2 et A. Un tel dispositif est, par exemple, une seringue à deux compartiments, voire trois compartiments.

Il est également possible que dans le dispositif de conditionnement, les compositions A et B1 soient conditionnées séparément l'une de l'autre, dans deux unités de conditionnement distinctes. Dans le cas où une composition C est présente, la composition C pourra également être conditionnée dans une unité de conditionnement distincte.

La description détaillée ci-après des compositions A, B1 et C permet de mieux comprendre l'invention.

### Composition A

La composition A contient du chitosane. Le chitosane, est un dérivé partiellement, voire totalement désacétylé de la chitine. Ses différentes formes sont notamment caractérisées par leur degré d'acétylation (DA) et par leur masse moléculaire moyenne en masse M_{w}.

De manière préférée, le chitosane de la composition A présente une masse moléculaire moyenne M_{w} appartenant à la gamme allant de 80 à 1000 kg/mol, de préférence, à la gamme allant de 100 à 700 kg/mol et/ou le chitosane présente un degré d'acétylation appartenant à la gamme allant de 0 à 60%, de préférence, à la gamme allant de 0 à 20%. Un degré d'acétylation inférieur ou égal à 20% est préféré, pour éviter le caractère inflammatoire du chitosane. On préférera donc utiliser un chitosane présentant une masse moléculaire moyenne M_{w} appartenant à la gamme allant de 100 à 700 kg/mol et un degré d'acétylation appartenant à la gamme allant de 0 à 20%.

Dans le cadre de l'invention, les masses moléculaires moyennes en masse M_{w} du chitosane sont déterminées par chromatographie d'exclusion stérique, dont les conditions expérimentales sont décrites dans la publication «Physico-chemical studies of the gelation of chitosan in a hydroalcoholic médium » A. MONTEMBAULT, C. VITON, A. DOMARD Biomaterials, 26(8), 933-943, 2005.

Le degré d'acétylation (DA) est déterminé en utilisant la technique de RMN du proton, en suivant la méthodologie d'Hirai (Asako Hirai, Hisashi Odani, Akio Nakajima, Polymer Bulletin (1991) Volume: 26, Issue: 1, Publisher: Springer, Pages: 87-94).

De manière avantageuse, le pH de la composition A appartient à la gamme allant de 4 à 6,5. Un tel pH permet que la suspension injectée, obtenue après mélange de la composition A et de celle comprenant les particules de verre(s) bioactif(s) et stabilisation, n'ait pas un pH inférieur à 6,5, voire même de préférence inférieur à 6,8, le pKa de l'amine des résidus glucosamine du chitosane étant de 6,2. Un pH plus bas de la solution A proche de 4 peut néanmoins être envisagé, si le temps entre le mélange de la composition A et de celle comprenant les particules de verre(s) bioactif(s) et l'injection de la composition injectable alors obtenue est suffisant pour permettre au pH de remonter au-dessus de 6,5.

En particulier, la composition A comprend une quantité d'acide nécessaire pour protoner les fonctions amine présentes sur le chitosane, ledit acide étant, de préférence, de l'acide acétique ou chlorhydrique.

Notamment, la composition A comprendra une quantité d'acide juste nécessaire pour protoner les fonctions amine des résidus glucosamine du chitosane. L'acide sera donc, de préférence, présent en quantité équimolaire par rapport aux résidus glucosamine du chitosane.

Dans la composition A, le chitosane sera donc sous une forme protonée, au niveau de ses fonctions amine, qui seront donc sous la forme -NH₃⁺. Ces groupements -NH₃⁺ créent des répulsions sur les chaînes de chitosane, qui vont alors se trouver sous forme déployée. Le polymère est donc dissout en solution aqueuse.

L'ajustement du pH de la solution aqueuse de chitosane pourra être effectué par incorporation d'acide acétique dans la solution aqueuse de chitosane ou par incorporation de soude. Un autre acide, tel que l'acide chlorhydrique, pourrait également être utilisé.

De manière préférée, la composition A comprend une concentration en chitosane de 0,5 à 6 % (m/m), de préférence de 1 à 4% (m/m). Cette concentration est exprimée en % m/m, à savoir le % que représente la masse de chitosane sur la masse de la composition A. Avec les concentrations en chitosane les plus faibles, la solution est moins visqueuse, ce qui permet de mieux contrôler l'homogénéité du gel final, après mélange avec les particules de verre(s) bioactif(s). Le gel obtenu *in situ* avec les plus faibles concentrations en chitosane est plus facilement colonisable par les cellules.

De manière particulièrement préférée, le pH de la composition A appartient à la gamme allant de 4 à 6,5 et elle contient une concentration en chitosane de 1 à 4% (m/m).

### Compositions B1 et B2

De manière avantageuse, la composition B1 comprend des particules d'un verre bioactif qui comprend au moins du SiO₂, du Na₂O, du CaO et du P₂O₅.

Bien d'autres types de verres pourront être utilisés. Le ou les verres seront sélectionnés de manière à obtenir, après mélange des compositions de chitosane et de verre(s) bioactif(s) sélectionnées, une remontée de pH pour le mélange, dans un temps donné. En particulier, il est cherché à obtenir pour la composition injectable, à un temps t qui appartient à la gamme allant de 5 à 20 minutes, un pH appartenant à la gamme allant de 6,8 à 7,4.

En particulier, ledit verre bioactif comprend 30% à 70% en poids de SiO₂, 10 à 50% en poids de CaO, 5 à 40% en poids de Na₂O et de 2 à 20% en poids de P₂O₅·

Notamment, un verre bioactif dont la composition est la suivante pourra être utilisé : 40 à 60 % en poids de SiO₂, 10 à 30 % en poids de CaO, 10 à 35 % en poids de Na₂O et 2 à 8 % en poids de P₂O₅. Un tel verre bioactif est le verre 45S5 (commercialisé notamment par Biometic ou Mo-Sci Corporation, ou sous forme de dispositifs médicaux par les sociétés Noraker, Novabone, etc.). Ce verre bioactif ne reste que modérément utilisé par les praticiens, principalement car sa mise en forme reste très limitée. La solution à base de chitosane décrite dans la présente invention va permettre, après gélification, de constituer un support matriciel pour les particules de verre bioactif, ce qui répond aux problèmes antérieurs.

Bien d'autres compositions de verres pourront être utilisées : l'important est que le verre bioactif comporte une quantité suffisante d'oxyde susceptible de relarguer des cations qui vont être capables de s'échanger avec les protons du milieu. Un tel échange doit pouvoir intervenir lorsque le verre bioactif est en suspension aqueuse, et en particulier, lorsqu'il est en mélange avec la solution de chitosane. De tels cations peuvent être des cations d'un métal alcalin ou alcalino-terreux, par exemple, des ions Ca²⁺, Na⁺, K⁺, Mg²⁺ Ba²⁺ ou Sr²⁺. La composition dudit verre bioactif comprendra donc au moins un des composants suivants : CaO, Na₂O, K₂O, MgO, ou SrO. La présence de strontium qui est un composant radio-opaque, permettra une localisation de la composition *in vivo,* après injection et gélification. Le verre comprendra également généralement du SiO₂. Cette part de SiO₂ sera, de préférence, sélectionnée de manière à ne pas empêcher la mobilité des cations au sein du verre bioactif qui ont pour vocation de venir s'échanger avec les protons du chitosane et ainsi autoriser leur relargage. En particulier, la composition B1 comprendra des particules d'un verre bioactif comprenant de 40 à 60% en poids de SiO₂ et 2 à 8% en poids de P₂O₅ et au moins un composant, voire 2, 3 ou 4, choisi(s) parmi Ca²⁺, Na⁺, K⁺, Mg²⁺, Sr²⁺, B³⁺ avec la totalité de ces composants qui représente de 30 à 55% en poids. De manière classique, la composition d'un verre bioactif est donnée dans le présent texte en % en poids, par rapport au poids total dudit verre et la somme des % de chacun des constituants donnés feront soit 100%, ou pour le moins au moins 95 %, ce qui n'exclut donc pas forcément la présence d'autres composants.

Le verre bioactif entraîne une augmentation du pH, lorsqu'il est mélangé à la solution aqueuse de chitosane (composition A). Cette augmentation de pH fait que les fonctions -NH₃⁺ initialement présentes sur les chaînes de chitosane ne vont plus se trouver sous forme protonée, mais sous forme -NH₂, permettant ainsi la formation de liaisons hydrogène et de liaisons hydrophobes entre les chaînes polymères, voire au sein d'une même chaîne, conduisant au final à une gélification.

Il est également possible d'utiliser une combinaison de plusieurs verres bioactifs : par exemple, un premier verre bioactif principalement utilisé pour l'obtention du pouvoir gélifiant et un deuxième verre bioactif principalement utilisé pour son pouvoir ostéoinducteur, ou qui va conduire au relargage d'ions, avec effet retard. La présence de SrO pour favoriser la localisation de l'implant pourra être prévue dans l'une ou l'autre des compositions de verre bioactif. De manière préférée, le premier verre bioactif pourra être un de ceux précédemment décrits, notamment un verre bioactif comprenant 40 à 60 % en poids de SiO₂, 10 à 30 % en poids de CaO, 10 à 35 % en poids de Na₂O et 2 à 8 % en poids de P₂O₅. Le deuxième verre bioactif pourra comprendre, quant à lui, 60% à 75% en poids de P₂O₅, 6 à 35% en poids de CaO, 5 à 12% en poids de Na₂O, 0 à 18% en poids de K₂O et 0 à 8% en poids de Al₂O₃.

De manière avantageuse, les particules de verre bioactif ont une taille qui appartient à une gamme allant de 3 à 500 µm, et notamment qui appartient à une gamme allant de 60 à 250 µm.

Bien entendu, la composition du verre bioactif ou du mélange de verre(s) bioactif(s) sélectionné(s) et la taille des particules dudit verre bioactif a une influence sur la bioactivité du produit et sur la gélification de la composition. Les particules de verres bioactifs pourront être obtenues par broyage de lingot de verres bioactifs et la taille souhaitée sera obtenue, par passage des particules obtenues sur des tamis, selon des techniques bien connues de l'homme du métier. La taille mentionnée dans le cadre de l'invention, à moins qu'il n'en soit spécifié autrement, correspond donc à la plus grande dimension d'une particule. Le choix d'une granulométrie plus fine entraîne une gélification plus rapide. Aussi, si la composition d'un verre bioactif contient moins de cations relargables, l'homme du métier pourra, en diminuant la taille des particules dudit verre bioactif, augmenter la quantité de cations relargués et donc obtenir l'effet souhaité vis-à-vis du chitosane.

La composition B1 se présentant sous la forme d'une poudre de particules d'un ou plusieurs verre(s) bioactif(s) pourra être directement mélangée à la composition A, pour former la formulation injectable. Il est également possible de former intermédiairement une suspension aqueuse de particules d'un ou plusieurs verre(s) bioactif(s) (composition B2). Lorsqu'une composition B2 se présentant sous la forme d'une suspension aqueuse de particules d'un ou plusieurs verre(s) bioactif(s) est formée intermédiairement, celle-ci sera, de préférence formée, peu de temps avant le mélange des compositions A et B2. En particulier, la suspension correspondant à la composition B2 sera formée, moins de 20 secondes, de préférence moins de 10 secondes, avant le mélange de cette dernière avec la composition A. En effet, le pH d'une suspension aqueuse de bioverres évolue en fonction du temps. Dans le cadre de l'invention, le pH d'une suspension B2 augmente avec le temps, du fait de l'échange de cations intervenant avec l'eau. Aussi, le pH de la composition B2 donné dans le cadre de l'invention, qui appartient à la gamme allant de 5,5 à 8, préférentiellement à la gamme allant de 6,8 à 7,2 correspond, de préférence, au pH de la suspension mesuré immédiatement après sa formation, notamment moins de 5 secondes, après sa constitution.

De manière avantageuse, la composition B2 comprend au moins 15% (m/m), de préférence de 30 à 60% de particules d'un ou plusieurs verre(s) bioactif(s). Ces % exprimés en % m/m, correspondent au % que représente la masse du ou des verres bioactifs présents sur la masse de la composition B2.

Lorsqu'une composition B2 correspondant à une suspension aqueuse d'un ou plusieurs verres bioactifs sera formée intermédiairement, elle sera obtenue, par introduction des particules d'un verre bioactifs ou d'un mélange de particules de verre bioactifs de la composition B1 dans la composition C. De manière préférée, la composition C est de l'eau, mais il n'est pas exclu qu'elle comprenne également des phosphates de calcium, sous réserve qu'elle respecte les conditions de pH qui doit appartenir à la gamme allant de 5,5 à 8, de préférence à la gamme allant de 6,8 à 7,2.

### Autres caractéristiques du produit selon l'invention et dispositif de conditionnement

Les solutions ou suspensions aqueuses utilisées dans la présente invention pour les compositions A, C et B2, seront physiologiquement acceptables. De préférence, de telles solutions ou suspensions seront réalisées dans de l'eau, et notamment de l'eau déionisée ou ultrapure.

Les proportions de composition A et de composition B1, voire de composition C quand celle-ci est présente, seront ajustées par l'homme du métier, notamment pour avoir la viscosité souhaitée. La masse de la composition B1 ou des compositions B1 et C, par rapport à la masse totale des compositions A et B1 ou des compositions A, B1 et C, respectivement, appartiendra à la gamme allant de 20% à 90%, de préférence à la gamme allant de 40% à 80%. La quantité des compositions B1 et C pourra être ajustée, par l'homme du métier, en fonction de la part massique que les particules de bioverre(s) représentent dans la composition B2 obtenues après leur mélange et ce, de manière à obtenir l'injectabilité souhaitée pour la composition finale. La quantité de verre bioactifs sera ajustée pour contrôler le temps nécessaire à l'obtention d'un gel. L'augmentation de la quantité de verre bioactif permet d'obtenir plus rapidement un pH plus élevé, et donc une gélification plus rapide.

Tous les paramètres précédemment listés seront ajustés par l'homme du métier, de manière à rendre compatible le temps nécessaire à la gélification avec le temps nécessaire pour l'injection. De préférence, la gélification interviendra entre 5 et 20 minutes après le mélange des compositions A et B1, ou des compositions A et B2, ou des compositions A, B1 et C, en fonction du produit selon l'invention et du mode de mélange sélectionné.

Selon un mode de réalisation particulier du produit selon l'invention, les compositions A et B1 sont conditionnées séparément l'une de l'autre dans deux compartiments d'un unique ensemble de conditionnement, et en particulier, dans un système d'injection tel qu'une seringue à deux compartiments. Lorsque le produit selon l'invention comprend également une composition C, de manière avantageuse, les compositions A, B1 et C sont conditionnées séparément les unes des autres dans trois compartiments d'un unique ensemble de conditionnement, et en particulier, dans un système d'injection tel qu'une seringue à trois compartiments.

Selon un autre mode de réalisation particulier du produit selon l'invention, les compositions A et B1 sont conditionnées séparément l'une de l'autre, dans deux unités de conditionnement distinctes. Lorsque le produit selon l'invention comprend également une composition C, de manière avantageuse, les compositions A, B1 et C sont conditionnées séparément les unes des autres dans trois unités de conditionnement distinctes.

Toutes les combinaisons sont possibles, la première étant néanmoins préférée :
- composition C et B1 dans un unique ensemble de conditionnement comprenant les compositions C et B1 conditionnées séparément l'une de l'autre dans deux compartiments dudit ensemble de conditionnement, et composition A conditionnée séparément dans une autre unité de conditionnement ;
- composition A et B1 dans un unique ensemble de conditionnement comprenant les compositions A et B1 conditionnées séparément l'une de l'autre dans deux compartiments dudit ensemble de conditionnement, et composition C conditionnée séparément dans une autre unité de conditionnement ;
- composition A et C dans un unique ensemble de conditionnement comprenant les compositions A et C conditionnées séparément l'une de l'autre dans deux compartiments dudit ensemble de conditionnement, et composition B1 conditionnée séparément dans une autre unité de conditionnement.

Dans ces différents cas, la composition B1 se présente sous une forme sèche, c'est-à-dire sous la forme d'une poudre de particules d'un ou plusieurs verres bioactifs. Avec une telle composition B1, et en particulier, une composition B1 constituée exclusivement de particules d'un ou plusieurs verres bioactifs, le produit selon l'invention est particulièrement stable.

Pour le reste, les caractéristiques préférées des compositions A et B1 précédemment listées s'appliquent, également de manière préférée, au dispositif de conditionnement selon l'invention.

En particulier, le dispositif de conditionnement selon l'invention combinera :
- une composition A présentant un pH qui appartient à la gamme allant de 4 à 6,5 et qui contient une concentration en chitosane de 1 à 4% (m/m),
- une composition B1 correspondant à des particules d'un ou plusieurs verre(s) bioactif(s), notamment sous la forme d'une poudre.

Les quantités de composition A et B1 seront sélectionnées, de manière à ce que, la masse de la composition B1, par rapport à la masse totale de la composition injectable obtenue, appartienne à la gamme allant de 20% à 90%, de préférence à la gamme allant de 40% à 80%.

Bien entendu, le chitosane et les bioverres correspondront, de manière avantageuse, à ceux présentés comme préférés dans les parties précédentes relatives aux compositions A et B1. Lorsque le dispositif de conditionnement comprend une composition C, celle-ci sera, de préférence de l'eau.

### Procédé de préparation de la composition injectable

L'invention a pour objet un procédé de préparation d'une composition injectable exempte de glycérophosphate contenant des particules d'un ou plusieurs verre(s) bioactif(s) en suspension dans une solution aqueuse de chitosane, ledit procédé comprenant, voire consistant exclusivement dans le mélange :
- d'une solution aqueuse de chitosane de pH appartenant à la gamme allant de 3 à 7, dite composition A et
- d'une poudre de particules d'un ou plusieurs verre(s) bioactif(s), dite composition B1,
   en l'absence de glycérophosphate.

L'invention a également pour objet un procédé de préparation d'une composition injectable, exempte de glycérophosphate, contenant des particules d'un ou plusieurs verre(s) bioactif(s) en suspension dans une solution aqueuse de chitosane, caractérisé en ce qu'il comprend, voire consiste exclusivement en les étapes successives suivantes :
- disposer d'une solution aqueuse de chitosane de pH appartenant à la gamme allant de 3 à 7, dite composition A,
- d'une poudre de particules d'un ou plusieurs verre(s) bioactif(s), dite composition B1,
- mettre la composition B1 en suspension aqueuse, et préférentiellement, en suspension dans de l'eau,
- mélanger la composition A et la suspension aqueuse de particules d'au moins un verre bioactif obtenue, rapidement après la mise en suspension de la composition B1, de préférence, moins de 20 secondes après, et préférentiellement, moins de 10 secondes après,
   les différentes compositions et solutions utilisées étant exemptes de glycérophosphate.

De manière préférée, la préparation de la composition injectable se fait par utilisation des compositions A et B1, des compositions A et B2, voire de compositions A, B1 et C, contenues dans un produit selon l'invention tel que précédemment défini.

De manière préférée, la composition injectable selon l'invention ne contient pas de génépine, ni de glutaraldéhyde, ni de formaldéhyde. Plus généralement, la composition injectable selon l'invention ne contient pas d'agent de réticulation. Aussi, de tels produits ne seront pas présents dans les différentes compositions et solutions utilisées dans les procédés selon l'invention.

Le mélange des compostions A et B1, des compositions A et B2, voire des compositions A, B1 et C, pour obtenir la composition injectable, pourra se faire par toute méthode appropriée d'addition ou d'injection notamment.

La composition A peut être directement mélangée à une composition B1 constituée d'une poudre de particules de verre(s) bioactif(s), par exemple, en mettant directement les particules de bioverre sous la forme d'une poudre dans la solution de chitosane. Il est également possible de mettre les particules de bioverre(s) en suspension aqueuse avant de procéder au mélange avec la solution de chitosane (composition A). Cette mise en suspension pourra se faire avec une composition C, présente dans le produit selon l'invention ou avec une solution aqueuse, et en particulier de l'eau, externe au produit selon l'invention. Dans les deux cas, cette mise en suspension sera réalisée juste avant de procéder au mélange avec la composition A, par exemple, moins de 20 secondes avant, et préférentiellement, moins de 10 secondes avant. Dans ce cas, le produit selon l'invention pourra comprendre trois compartiments, disposé dans un ensemble unique de conditionnement ou dans plusieurs unités de conditionnement : l'un contenant la composition A, l'autre la composition B1 de verre(s) bioactif(s) et le troisième de l'eau (composition C).

Selon une première variante de mise en œuvre, le procédé de préparation d'une composition injectable selon l'invention comprend, voire consiste exclusivement en les étapes successives suivantes :
- disposer d'une composition A, telle que définie dans le cadre de l'invention,
- disposer d'une composition B1,
- mélanger les compositions A et B1.

En particulier, selon une deuxième variante de mise en œuvre, le procédé de préparation d'une composition injectable selon l'invention comprend, voire consiste exclusivement en les étapes successives suivantes :
- disposer d'une composition A, telle que définie dans le cadre de l'invention,
- disposer d'une composition B1,
- mettre la composition B1 en suspension aqueuse, et préférentiellement, en suspension dans de l'eau,
- mélanger les compositions A et la suspension obtenue, rapidement après la mise en suspension de la composition B1, de préférence, moins de 20 secondes après, et préférentiellement, moins de 10 secondes après.

La suspension de verres formée comprendra, de préférence, au moins 15% (m/m), de préférence de 30 à 60% de particules d'un ou plusieurs verre(s) bioactif(s). De manière avantageuse, la mise en suspension de la composition B1 correspond à la formation d'une composition B2, présentant un pH qui appartient à la gamme allant de 5,5 à 8, préférentiellement à la gamme allant de 6,8 à 7,2, lorsque ce pH est mesuré immédiatement après sa formation, notamment moins de 5 secondes après sa constitution.

De manière préférée, la suspension formée est obtenue par mise en suspension de la composition A, dans une composition C telle que définie dans le cadre de l'invention.

Dans de tels procédés, le mélange peut être rendu plus homogène grâce à une agitation mécanique ou grâce à un mélange dynamique (flux dynamique d'une composition dans l'autre, notamment de la composition A dans la composition B1, ou inversement, ou de la suspension B2 dans la composition A notamment).

Là encore, les caractéristiques préférées des compositions A et B1 précédemment listées s'appliquent, également de manière préférée, au procédé de préparation selon l'invention.

En particulier, seront utilisées dans le procédé selon l'invention :
- une composition A présentant un pH qui appartient à la gamme allant de 4 à 6,5 et qui contient une concentration en chitosane de 1 à 4% (m/m),
- une composition B1 correspondant à des particules d'un ou plusieurs verre(s) bioactif(s), notamment sous la forme d'une poudre.

Les quantités de composition A et B1, voire les quantités de solution aqueuse et en particulier d'eau pour réaliser une suspension intermédiaire, seront sélectionnées, de manière à ce que la masse de la composition B1, par rapport à la masse totale de la composition injectable obtenue, appartienne à la gamme allant de 20% à 90%, de préférence à la gamme allant de 40% à 80%.

Bien entendu, là encore, le chitosane et les bioverres correspondront, de manière avantageuse, à ceux présentés comme préférés dans les parties précédentes relatives aux compositions A et B1.

L'invention a également pour objet les compositions injectables susceptibles d'être obtenues par les procédés de préparation décrits dans le cadre de l'invention.

La composition obtenue est destinée à être injectée à un sujet. De préférence, le mélange des compositions A et B1, voire des compositions B1 et C formant une composition B2, puis des compositions A et B2, ou encore des compositions A, B1 et C, aura lieu juste avant l'injection de la composition injectable obtenue à un sujet, notamment moins de 1 minute avant, de préférence moins de 30 secondes avant. Bien entendu, l'injection devra intervenir avant la gélification.

Les exemples ci-après, en référence aux Figures annexées, permettent d'illustrer l'invention mais n'ont aucun caractère limitatif.
La **Figure 1** présente l'évolution des modules G' et G" en fonction de la fréquence pour une solution de chitosane de concentration 0,375 % m/v (déformation imposée : 0,5 %).
La **Figure 2** montre l'évolution de G' en G" en fonction de la fréquence angulaire, 2 minutes 30 secondes après la réalisation du mélange (déformation imposée : 0,5 %).
La **Figure 3** présente le diffractogramme aux rayons X obtenu après immersion d'un hydrogel formé à partir d'une suspension contenant des bioverres et du chitosane, pendant 4 jours dans du SBF à 37°C.

### Méthode de détermination du pH

La valeur du pH du mélange des compositions est avantageusement contrôlée grâce à un pH-mètre de contact (Mettler-Toledo AG, Suisse).

### Méthode de détermination de la viscosité

La mesure de la viscosité est réalisée à haute vitesse de cisaillement (typiquement 10⁴ s⁻¹) et est obtenue par rhéométrie capillaire selon la méthode décrite dans la publication « Chitosan solutions as injectable systems for dermal filler applications: rheological characterization and biological evidence » par C. Halimi, A. Montembault, A. Guerry, T. Delair, E. Viguier, R. Fulchiron, L. David. Annual International Conference of the IEEE Engineering in Medicine and Biology Society. IEEE Engineering in Medicine and Biology Society. 2015; 2596-9.

### Méthode d'ajustement de la granulométrie (taille des particules) des bioverres

Les verres obtenus sont broyés à l'aide d'un mortier/pilon en Agathe ou à l'aide d'un broyeur à billes en carbure de tungstène.

Les poudres obtenues sont ensuite tamisées à sec à travers une colonne de tamis vibrants de diamètres de maille variables (63µm, 90µm, 125µm, 150µm, 200µm, 250µm, 315µm et/ou 500µm).

Afin d'éliminer les fines particules agglomérées qui ne correspondent pas aux granulométries recherchées, les différents lots obtenus après séparation par tamisage sont plongés chacun dans un bêcher contenant 200ml d'acétone et placé dans un bac à ultrasons. Les grosses particules tombent au fond du bêcher, alors que les fines sont éliminées avec l'acétone. Cette procédure est répétée jusqu'à ce que l'acétone soit claire (exempte de fines particules). Les lots de poudre de granulométrie définie sont ensuite séchés dans une étuve à 92°C pendant 20 à 30 minutes.

### Méthode d'obtention du diffractogramme

La mesure des diffractogrammes est réalisée à l'aide d'un diffractomètre à rayons X de laboratoire, utilisant une source de rayons X Cu Kα et des angles de diffraction compris entre 3 et 60 degrés 2θ (soit des modules du vecteur de diffusion q compris entre 0,2 et 4), et en géométrie Bragg-Brentano.

### 1. Mise en évidence de la formation d'un gel

Une solution aqueuse de chitosane à 0,75% en masse est préparée. Pour cela, le chitosane est dispersé dans un volume d'eau déionisée. De l'acide acétique est ajouté, de façon à permettre une protonation stœchiométrique des fonctions amine du chitosane. Le chitosane utilisé dans le cas de cet exemple possède les caractéristiques structurales suivantes : degré d'acétylation de 5%, masse molaire *M_{w}* de 550 000 g/mol et un indice de polydispersité *Iₚ* de 1,4.

Parallèlement, une poudre de verre bioactif est dispersée dans de l'eau déionisée, dont le volume correspond au volume de la solution de chitosane. Plus exactement, il s'agit du verre 45S5 dont la composition en masse est la suivante : 45% de SiO₂, 24,5% de CaO, 24,5% de Na₂O et 6% de P₂O₅. La masse de verre incorporée est égale à la moitié de la masse correspondant à la solution de chitosane.

Ainsi, les quantités utilisées dans cet exemple sont les suivantes :
- 750 mg de solution de chitosane à 0,75% (m/m),
- 375 mg de particules de verre 45S5 dispersées dans 750 mg d'eau déionisée.

Les verres utilisés et présentés dans le cadre de cette invention ont été préparés au Laboratoire Charles Coulomb de l'Université de Montpellier. Les bioverres ont été élaborés à partir d'un mélange des matières premières suivantes : Al(OH)₃, H₂NaPO₄, CaCO₃, H₂(NH)₄PO₄, SiO₂ et K₂CO₃ en proportions adéquates en fonction de la composition souhaitée. Dans le cas de l'élaboration du verre 45S5, les matières premières utilisées sont Na₂CO₃ (Prolabo), CaCO₃ (MerckKGaA), H₂(NH)₄PO₄ (Prolabo), et SiO₂ (Prolabo). Chaque mélange a été fondu dans un creuset en platine. La fonte obtenue a été coulée dans un moule en acier et le verre obtenu après refroidissement à l'air a été recuit 1 h à une température un peu supérieure à sa température de transition vitreuse, puis refroidi à une vitesse très lente jusqu'à température ambiante.

Chaque lingot de verre formé a ensuite été broyé et tamisé, afin d'obtenir des poudres de granulométries différentes. La granulométrie de la poudre de verre utilisée dans le cadre de cet exemple est comprise entre 200 et 315 microns et est sélectionnée grâce aux tamis utilisés.

La formulation associant la dispersion de bioverre et la solution de chitosane a été mise sous agitation, de façon à obtenir un système homogène. Au final, la concentration de chitosane dans le mélange est de 0,375 % (m/v).

Lors de la réalisation du mélange sous agitation, un mécanisme de gélification spontanée se produit conduisant à la formation d'un hydrogel physique contenant une partie minérale vitreuse.

Quinze minutes après le mélange, le pH du matériau composite se stabilise à 7,4.

### 2. Caractéristiques rhéologiques

Cette partie présente les caractérisations rhéologiques qui ont été réalisées, afin de mettre en évidence le pouvoir gélifiant des bioverres lors de leur mélange avec une solution de chitosane.

Une solution aqueuse de chitosane à 0,75% en masse a été préparée, comme précédemment.

Parallèlement, des verres bioactifs sous la forme de poudre sont dispersés dans de l'eau déionisée dont le volume est égal au volume de la solution de chitosane. Plus exactement, il s'agit d'un mélange de verres composé de 2/3 de 45S5 et d'1/3 de verre Na₁₆Ca₃₀P₅₀Al₄ (en proportions massiques). Le verre Na₁₆Ca₃₀P₅₀Al₄ (appelé ici « verres au phosphate ») est composé de 9,8% de Na₂O, 16,5% de CaO, 69,7% de P₂O₅ et de 4% d'Al₂O₃ (% m/m). Ce verre a été élaboré en suivant la même procédure que celle décrite précédemment dans le paragraphe **1. Mise en évidence de la formation d'un gel,** c'est-à-dire à partir d'un mélange de matières premières qui sont ici : Al(OH)₃ (FlukaBioChemika), H₂NaPO₄ (FlukaChemie) CaCO₃ (MerckKGaA), H₂(NH)₄PO₄ (Prolabo) en proportions adéquates, fondues dans un creuset platine. Le verre obtenu par coulage de la fonte suivi d'une recuisson et d'un refroidissement lent a ensuite été broyé et tamisé, comme cela a été décrit pour la composition A au paragraphe Méthode d'ajustement de la granulométrie (taille des particules) des bioverres. La granulométrie des poudres des verres 45S5 et Na₁₆Ca₃₀P₅₀Al₄ utilisées dans le cadre de cet exemple est comprise entre 90 et 150 microns.

La masse des verres incorporée est égale à la moitié de la masse de la solution de chitosane.

Ainsi, les quantités utilisées dans cet exemple sont les suivantes :
- 750 mg de solution de chitosane à 0,75 % (m/m),
- 375 mg de verres dispersés dans 750 mg d'eau déionisée.

Dans ces 375 mg sont présents 250 mg de verre 45S5 et 125 mg de verre au phosphate.

La granulométrie des verres utilisés dans le cadre de cet exemple est comprise entre 90 et 150 microns et est sélectionnée grâce aux tamis utilisés.

La formulation associant la dispersion de bioverres et la solution de chitosane est mise sous agitation de façon à obtenir un système homogène. Au final, la concentration en chitosane dans le mélange est de 0,375% (m/v).

Afin de faciliter l'homogénéisation de la formulation, un système de deux seringues connectées est utilisé. Un connecteur relie les deux seringues, l'une contenant la solution de chitosane, l'autre la suspension de bioverres. Le mélange est ainsi réalisé en effectuant plusieurs allers-retours entre les deux seringues.

Lors de la réalisation du mélange homogène, un mécanisme de gélification spontanée se produit conduisant à la formation d'un hydrogel physique contenant une partie minérale vitreuse.

La transition sol-gel correspondante a été étudiée par rhéométrie.

Ces essais ont été réalisés sur un rhéomètre AR 2000 (TA Instruments), en utilisant une géométrie plan-plan avec un plateau de 25 mm de diamètre et un système de confinement de l'échantillon permettant de maintenir une atmosphère saturée en eau et ainsi de limiter l'évaporation de l'eau présente dans l'échantillon. L'ensemble des essais a été réalisé à température ambiante (25°C).

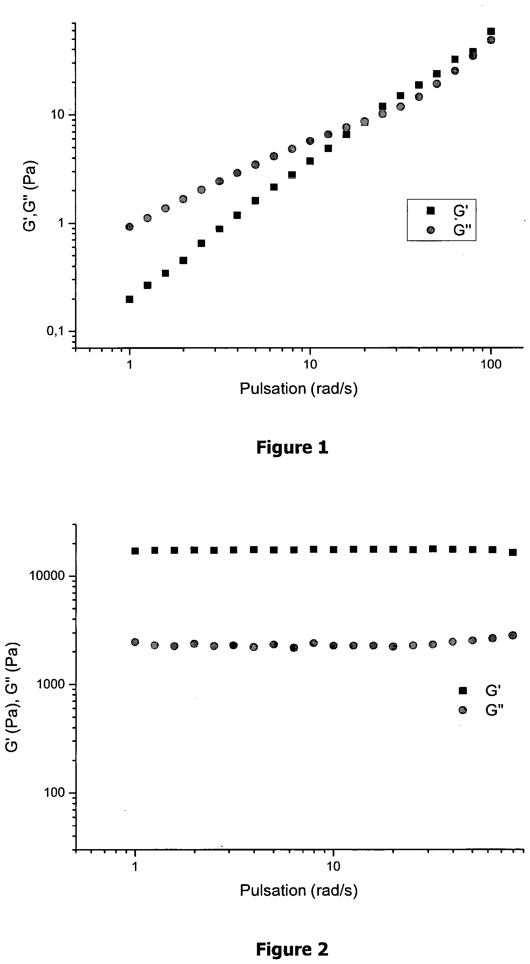

Une caractérisation rhéologique a été d'abord réalisée sur une solution de chitosane concentrée à 0,375% (m/v) ne contenant pas de verre. Les courbes obtenues pour une formulation sans verre sont présentées sur la **Figure 1** et montrent un comportement rhéologique correspondant à l'état de solution. L'allure des courbes est caractéristique des solutions viscoélastiques avec G'<G" aux basses fréquences et G'>G" aux hautes fréquences, G' et G" étant respectivement définis comme étant les modules élastique et visqueux de l'échantillon.

Une caractérisation rhéologique a ensuite été réalisée sur une même solution, mais dans laquelle a été incorporée une suspension de particules de bioverres dispersées dans l'eau déionisée (suspension de verre 45S5/verre au phosphate et introduite dans la proportion précédemment décrite).

La **Figure 2** montre les courbes rhéologiques obtenues 2 minutes 30 secondes après la réalisation du mélange. Ces courbes montrent un comportement rhéologique correspondant à un état de gel, avec des valeurs de modules G' et G" telles que G' ≥ 10 G", quelle que soit la fréquence angulaire.

Cette propriété de gélification est tout à fait compatible avec le développement de systèmes injectables.

La présence des bioverres au sein de la solution provoque une augmentation du pH dans le milieu et induit la déprotonation des sites amine du chitosane, ce qui permet la gélification de la solution de polymère.

### 3. Etude de révolution des gels obtenus au contact d'une solution dont la composition est proche de celle des fluides physiologiques humains (solution « Simulated Body Fluid »)

Une étude a été réalisée, afin de vérifier que lorsque les particules de bioverres sont présentes dans l'hydrogel, les bioverres préservent leurs propriétés de bioactivité.

Afin d'évaluer cette bioactivité, les gels élaborés ont été immergés dans une solution SBF (« Simulated Body Fluid »). Cette solution SBF possède une concentration d'ions proche de celle des fluides physiologiques humains maintenus dans des conditions de température et de pH physiologiques (37°C et 7,5) [T. Kokubo, H.K., S. Sakka, T. Kitsugi and T. Yamamuro. Solutions able to reproduce in vivo surface-structure changes in bioactive glass-ceramic A-W J. Biomed. Mater. Res. 1990, vol. 24, 721-734].

Les composites étudiés ici avaient la composition suivante :
- 750 mg de solution de chitosane à 0,75 % (m/m) dans de l'eau déionisée,
- 375 mg de verres dispersés dans 750 mg d'eau déionisée, ces 375 mg comprenant 250 mg de verre 45S5 et 125 mg de verre au phosphate décrits dans la partie précédente 2.

La granulométrie des verres utilisés dans le cadre de cet exemple était comprise entre 90 et 150 microns.

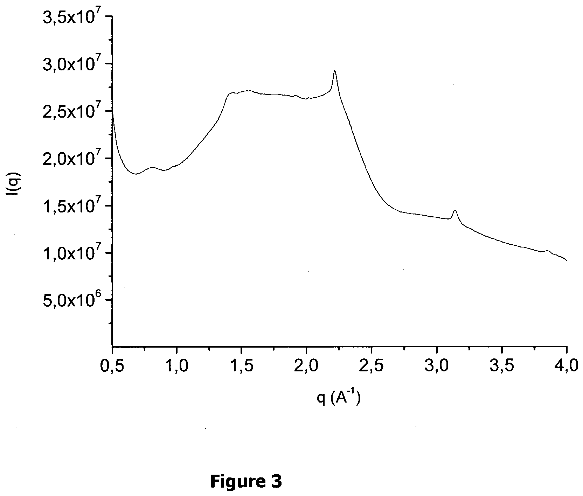

Ainsi, après immersion des matériaux composites dans une solution SBF pendant deux jours, l'eau de l'échantillon obtenu a été éliminée par lyophilisation, et analysée par diffraction des rayons X. Le diffractogramme aux rayons X, présenté sur la **Figure 3** montre la formation d'une phase cristalline, la calcite. Les flèches pointent les pics de diffraction caractéristiques de la calcite. Dans le cas des bioverres seuls, il est généralement observé que la calcite qui précipite, dans un premier temps, se transforme ensuite en hydroxyapatite : cette phase peut être ainsi considérée dans ces conditions comme un précurseur de l'hydroxyapatite carbonatée [Lefebvre, L., Développement de bioverres poreux pour application à l'orthopédie et à l'ingénierie tissulaire. 2007, INSA de Lyon].

Dans le cas des bioverres incorporés dans l'hydrogel, les phénomènes d'échanges ioniques avec le milieu sont donc comparables à ce qui est observé dans le cas de bioverres seuls. Ces expériences montrent que le comportement des bioverres n'a pas été modifié dans le cas de leur incorporation dans un hydrogel de chitosane et que ces bioverres ont conservé leurs propriétés de bioactivité.

## Revendications

1. Produit destiné à la préparation d'une composition injectable contenant de manière séparée l'une de l'autre, au moins une première et une deuxième compositions, et éventuellement une troisième composition C et dans lequel :
- la première composition dite A est une solution aqueuse de chitosane de pH appartenant à la gamme allant de 3 à 7, le chitosane présentant une masse moléculaire moyenne M_{w} appartenant à la gamme allant de 80 à 1000 kg/mol et un degré d'acétylation appartenant à la gamme allant de 0 à 60%,
- la deuxième composition dite B1 correspond à des particules d'un ou plusieurs verre(s) bioactif(s), et
- la troisième composition C lorsqu'elle est présente correspond à de l'eau ou à une solution aqueuse de pH appartenant à la gamme allant de 5,5 à 8 ;
ledit produit ne contenant pas de glycérophosphate et
la masse de la composition B1 ou des compositions B1 et C, par rapport à la masse totale des compositions A et B1, ou des compositions A, B1 et C, respectivement, appartient à la gamme allant de 20% à 90%.

2. Produit selon la revendication 1, **caractérisé en ce que** le pH de la composition A appartient à la gamme allant de 4 à 6,5.

3. Produit selon l'une quelconque des revendications 1 ou 2, **caractérisé en ce que** la composition A comprend une quantité d'acide nécessaire pour protoner les fonctions amine présentes sur le chitosane, ledit acide étant, de préférence, de l'acide acétique ou chlorhydrique.

4. Produit selon l'une quelconque des revendications 1 à 3, **caractérisé en ce que** la composition A comprend une concentration en chitosane de 0,5 à 6 % (m/m), de préférence de 1 à 4% (m/m).

5. Produit selon l'une quelconque des revendications 1 à 4, **caractérisé en ce que** la composition B1 comprend des particules d'un verre bioactif qui comprend au moins du SiO₂, du Na₂O, du CaO et du P₂O₅.

6. Produit selon l'une quelconque des revendications 1 à 5, **caractérisé en ce que** la composition B1 comprend des particules d'un premier verre bioactif comprenant 40 à 60 % en poids de SiO₂, 10 à 30 % en poids de CaO, 10 à 35 % en poids de Na₂O et 2 à 8 % en poids de P₂O₅ et des particules d'un deuxième verre bioactif comprenant 60% à 75% en poids de P₂O₅, 6 à 35% en poids de CaO, 5 à 12% en poids de Na₂O, 0 à 18% en poids de K₂O et 0 à 8% en poids de Al₂O₃.

7. Produit selon l'une quelconque des revendications 1 à 6, **caractérisé en ce que** les particules de verre bioactif ont une taille qui appartient à une gamme allant de 3 à 500 µm, et notamment qui appartient à une gamme allant de 60 à 250 µm.

8. Produit selon l'une quelconque des revendications 1 à 7, **caractérisé en ce qu'**il comprend également une composition C correspondant à de l'eau ou à une solution aqueuse de pH appartenant à la gamme allant de 5,5 à 8, de préférence à la gamme allant de 6,8 à 7,2.

9. Produit selon la revendication 8, **caractérisé en ce que** la composition C est présente en quantité telle que la composition B1 représente au moins 15% (m/m), de préférence de 30 à 60% (m/m), de la masse totale des compositions B1 et C.

10. Produit selon l'une quelconque des revendications 1 à 9, **caractérisé en ce que** la masse de la composition B1 ou des compositions B1 et C, par rapport à la masse totale des compositions A et B1, ou des compositions A, B1 et C, respectivement, appartient à la gamme allant de 40% à 60%.

11. Produit selon l'une quelconque des revendications 1 à 10, **caractérisé en ce que** le chitosane présente une masse moléculaire moyenne M_{w} appartenant à la gamme allant de 100 à 700 kg/mol.

12. Produit selon l'une quelconque des revendications 1 à 11, **caractérisé en ce que** le chitosane présente un degré d'acétylation appartenant à la gamme allant de 0 à 20%.

13. Produit selon l'une quelconque des revendications 1 à 12, **caractérisé en ce que** les compositions A et B1 sont conditionnées séparément l'une de l'autre dans deux compartiments d'un unique ensemble de conditionnement, et en particulier, dans un système d'injection tel qu'une seringue à deux compartiments.

14. Produit selon l'une quelconque des revendications 1 à 12, **caractérisé en ce que** les compositions A et B1 sont conditionnées séparément l'une de l'autre, dans deux unités de conditionnement distinctes.

15. Procédé de préparation d'une composition injectable, exempte de glycérophosphate, contenant des particules d'un ou plusieurs verre(s) bioactif(s) en suspension dans une solution aqueuse de chitosane, **caractérisé en ce qu'**il comprend, voire consiste exclusivement en les étapes successives suivantes :
- disposer d'une solution aqueuse de chitosane, dite composition A, ,
- d'une poudre de particules d'un ou plusieurs verre(s) bioactif(s), dite composition B1,
- mettre la composition B1 en suspension aqueuse dans une composition C,
- mélanger la composition A et la suspension aqueuse de particules d'au moins un verre bioactif obtenue, rapidement après la mise en suspension de la composition B1, de préférence, moins de 20 secondes après, et préférentiellement, moins de 10 secondes après,
les différentes compositions A, B1 et C étant définie selon les revendications 1 à 12 et étant exemptes de glycérophosphate.

16. Composition injectable correspondant à une suspension d'un ou plusieurs verre(s) bioactif(s) dans une solution aqueuse de chitosane, obtenue par mélange des compositions A et B1, par mélange des compositions A, B1 et C, ou par mélange des compositions B1 et C pour former une composition intermédiaire B2 puis B2 et C, du produit tel que défini à l'une quelconque des revendications 1 à 12.

17. Composition injectable correspondant à une suspension d'un ou plusieurs verre(s) bioactif(s) dans une solution aqueuse de chitosane, susceptible d'être obtenu par un procédé défini à l'une quelconque des revendications 15 ou 16.

18. Composition injectable selon l'une quelconque des revendications 16 ou 17, pour son utilisation dans la régénération d'un tissu osseux, après injection à un sujet.

19. Composition injectable pour son utilisation selon la revendication 18, dans laquelle après injection à un sujet, il y a formation d'un hydrogel de chitosane *in vivo.*

20. Dispositif de conditionnement d'un produit selon l'une quelconque des revendications 1 à 12, ledit dispositif comprenant au moins deux compartiments indépendants contenant respectivement chacune desdites compositions A et B1.

## Patentansprüche

1. Produkt, das zur Herstellung einer injizierbaren Zusammensetzung bestimmt ist, enthaltend separat voneinander mindestens eine erste und eine zweite Zusammensetzung und eventuell eine dritte Zusammensetzung C und wobei:
- die erste Zusammensetzung A eine wässrige Chitosanlösung mit einem pH ist, der zu dem Bereich gehört, der von 3 bis 7 reicht, wobei das Chitosan eine mittlere Molekularmasse M_{w} aufweist, die zu dem Bereich gehört, der von 80 bis 1000 kg/mol reicht, und einen Acetylierungsgrad, der zu dem Bereich gehört, der von 0 bis 60 % reicht,
- die zweite Zusammensetzung B1 Partikeln von einem oder mehreren biologisch aktiven Glas/Gläsern entspricht, und
- die dritte Zusammensetzung C, wenn sie vorhanden ist, Wasser oder einer wässrigen Lösung mit einem pH entspricht, der zu dem Bereich gehört, der von 5,5 bis 8 reicht;
wobei das Produkt kein Glycerophosphat enthält, und
die Masse der Zusammensetzung B1 oder der Zusammensetzungen B1 und C in Bezug auf die Gesamtmasse jeweils der Zusammensetzungen A und B1 oder der Zusammensetzungen A, B1 und C zu dem Bereich gehört, der von 20 % bis 90 % reicht.

2. Produkt nach Anspruch 1, **dadurch gekennzeichnet, dass** der pH der Zusammensetzung A zu dem Bereich gehört, der von 4 bis 6,5 reicht.

3. Produkt nach einem der Ansprüche 1 oder 2, **dadurch gekennzeichnet, dass** die Zusammensetzung A eine Säuremenge umfasst, die notwendig ist, um die auf dem Chitosan vorhandenen Aminfunktionen zu protonieren, wobei die Säure vorzugsweise Essigsäure oder Salzsäure ist.

4. Produkt nach einem der Ansprüche 1 bis 3, **dadurch gekennzeichnet, dass** die Zusammensetzung A eine Chitosankonzentration von 0,5 bis 6 % (m/m), vorzugsweise von 1 bis 4 % (m/m) umfasst.

5. Produkt nach einem der Ansprüche 1 bis 4, **dadurch gekennzeichnet, dass** die Zusammensetzung B1 Partikel eines biologisch aktiven Glases umfasst, das mindestens SiO₂, Na₂O, CaO und P₂O₅ umfasst.

6. Produkt nach einem der Ansprüche 1 bis 5, **dadurch gekennzeichnet, dass** die Zusammensetzung B1 Partikel eines ersten biologisch aktiven Glases umfasst, das 40 bis 60 Gew.-% SiO₂, 10 bis 30 Gew.-% CaO, 10 bis 35 Gew.-% Na₂O und 2 bis 8 Gew.-% P₂O₅ umfasst, und Partikel eines zweiten biologisch aktiven Glases, das 60 bis 75 Gew.-% P₂O₅, 6 bis 35 Gew.-% CaO, 5 bis 12 Gew.-% Na₂O, 0 bis 18 Gew.-% K₂O und 0 bis 8 Gew.-% Al₂O₃ umfasst.

7. Produkt nach einem der Ansprüche 1 bis 6, **dadurch gekennzeichnet, dass** die biologisch aktiven Glaspartikel eine Größe haben, die zu einem Bereich gehört, der von 3 bis 500 µm reicht und der vor allem zu einem Bereich gehört, der von 60 bis 250 µm reicht.

8. Produkt nach einem der Ansprüche 1 bis 7, **dadurch gekennzeichnet, dass** es ebenfalls eine Zusammensetzung C umfasst, die Wasser oder einer wässrigen Lösung mit einem pH entspricht, der zu dem Bereich gehört, der von 5,5 bis 8 reicht, vorzugsweise zu dem Bereich, der von 6,8 bis 7,2 reicht.

9. Produkt nach Anspruch 8, **dadurch gekennzeichnet, dass** die Zusammensetzung C in einer Menge vorhanden ist, die derart ist, dass die Zusammensetzung B1 mindestens 15 % (m/m), vorzugsweise 30 bis 60 % (m/m), der Gesamtmasse der Zusammensetzungen B1 und C darstellt.

10. Produkt nach einem der Ansprüche 1 bis 9, **dadurch gekennzeichnet, dass** die Masse der Zusammensetzung B1 oder der Zusammensetzungen B1 und C in Bezug auf die Gesamtmasse jeweils der Zusammensetzungen A und B1 oder der Zusammensetzungen A, B1 und C zu dem Bereich gehört, der von 40 % bis 60 % reicht.

11. Produkt nach einem der Ansprüche 1 bis 10, **dadurch gekennzeichnet, dass** das Chitosan eine mittlere Molekularmasse M_{w} aufweist, die zu dem Bereich gehört, der von 100 bis 700 kg/mol reicht.

12. Produkt nach einem der Ansprüche 1 bis 11, **dadurch gekennzeichnet, dass** das Chitosan einen Acetylierungsgrad aufweist, der zu dem Bereich gehört, der von 0 bis 20 % reicht.

13. Produkt nach einem der Ansprüche 1 bis 12, **dadurch gekennzeichnet, dass** die Zusammensetzungen A und B1 getrennt voneinander in zwei Kammern einer einzigen Verpackungseinheit und insbesondere in einem Injektionssystem wie einer Spritze mit zwei Kammern verpackt sind.

14. Produkt nach einem der Ansprüche 1 bis 12, **dadurch gekennzeichnet, dass** die Zusammensetzungen A und B1 getrennt voneinander in zwei verschiedenen Verpackungseinheiten verpackt sind.

15. Verfahren zur Herstellung einer injizierbaren Zusammensetzung ohne Glycerophosphat, enthaltend Partikel eines oder mehrerer biologisch aktiven/aktiver Glases/Gläser in Suspension in einer wässrigen Chitosanlösung, **dadurch gekennzeichnet, dass** es die folgenden aufeinanderfolgenden Schritte umfasst, ja sogar ausschließlich daraus besteht:
- Bereitstellen einer wässrigen Chitosanlösung, bezeichnet als Zusammensetzung A,
- eines Pulvers aus Partikeln eines oder mehrerer biologisch aktiven/aktiver Glases/Gläser, bezeichnet als Zusammensetzung B1,
- Versetzen der Zusammensetzung B1 in wässrige Suspension in einer Zusammensetzung C,
- schnelles Mischen der Zusammensetzung A und der erhaltenen wässrigen Partikelsuspension mindestens eines biologisch aktiven Glases nach dem Versetzen in Suspension der Zusammensetzung B1 vorzugsweise weniger als 20 Sekunden später und vorzugsweise weniger als 10 Sekunden später,
wobei die verschiedenen Zusammensetzungen A, B1 und C nach den Ansprüchen 1 bis 12 und ohne Glycerophosphat sind.

16. Injizierbaren Zusammensetzung, die einer Suspension eines oder mehrerer biologisch aktiven/aktiver Glases/Gläser in einer wässrigen Chitosanlösung entspricht, erhalten durch Mischen der Zusammensetzungen A und B1, durch Mischen der Zusammensetzungen A, B1 und C oder durch Mischen der Zusammensetzungen B1 und C, um eine Übergangszusammensetzung B2, dann B2 und C eines Produkts nach einem der Ansprüche 1 bis 12 zu bilden.

17. Injizierbaren Zusammensetzung, die einer Suspension eines oder mehrerer biologisch aktiven/aktiver Glases/Gläser in einer wässrigen Chitosanlösung entspricht, die durch ein Verfahren nach einem der Ansprüche 15 oder 16 erhaltbar ist.

18. Injizierbaren Zusammensetzung nach einem der Ansprüche 16 oder 17 für ihre Verwendung bei der Regenerierung von Knochengewebe nach Injektion in einen Menschen.

19. Injizierbaren Zusammensetzung für ihre Verwendung nach Anspruch 18, wobei sich nach der Injektion in einen Menschen ein Chitosan-Hydrogel *in vivo* bildet.

20. Verpackungsvorrichtung eines Produkts nach einem der Ansprüche 1 bis 12, wobei die Vorrichtung mindestens zwei unabhängige Kammern umfasst, die jeweils jede der Zusammensetzungen A und B1 enthalten.

## Claims

1. A product intended for the preparation of an injectable composition containing separately from each other, at least a first and a second composition, and optionally a third composition C and wherein:
- the first composition called A is an aqueous solution of chitosan with a pH in the range of 3 to 7, the chitosan having an average molecular weight M_{w} in the range of 80 to 1000 kg/mol and a degree of acetylation in the range of 0 to 60%,
- the second composition called B1 corresponds to particles of one or more bioactive glass(es), and
- the third composition C when present corresponds to water or to an aqueous solution with a pH in the range of 5.5 to 8;
said product not containing glycerophosphate and
the mass of composition B1 or of compositions B1 and C, relative to the total mass of compositions A and B1, or of compositions A, B1 and C, respectively, is in the range of 20% to 90%.

2. The product as claimed in claim 1, **characterized in that** the pH of composition A is in the range of 4 to 6.5.

3. The product as claimed in any one of claims 1 or 2, **characterized in that** composition A comprises an amount of acid necessary to protonate the amine functions present on chitosan, said acid preferably being acetic or hydrochloric acid.

4. The product as claimed in any one of claims 1 to 3, **characterized in that** composition A comprises a chitosan concentration of 0.5 to 6% (m/m), preferably 1 to 4% (m/m).

5. The product as claimed in any one of claims 1 to 4, **characterized in that** composition B1 comprises particles of a bioactive glass which comprises at least SiO₂, Na₂O, CaO and P₂O₅.

6. The product as claimed in any one of claims 1 to 5, **characterized in that** the composition B1 comprises particles of a first bioactive glass comprising 40 to 60% by weight SiO₂, 10 to 30% by weight CaO, 10 to 35% by weight Na₂O and 2 to 8% by weight P₂O₅ and particles of a second bioactive glass comprising 60% to 75% by weight P₂O₅, 6 to 35% by weight CaO, 5 to 12% by weight Na₂O, 0 to 18% by weight K₂O and 0 to 8% by weight Al₂O₃.

7. The product as claimed in any one of claims 1 to 6, **characterized in that** the bioactive glass particles have a size in the range of 3 to 500 µm, and in particular in the range of 60 to 250 µm.

8. The product as claimed in any one of claims 1 to 7, **characterized in that** it also comprises a composition C corresponding to water or to an aqueous solution with a pH in the range of 5.5 to 8, preferably in the range of 6.8 to 7.2.

9. The product as claimed in claim 8, **characterized in that** composition C is present in such an amount that composition B1 represents at least 15% (m/m), preferably from 30 to 60% (m/m), of the total mass of compositions B1 and C.

10. The product as claimed in any one of claims 1 to 9, **characterized in that** the mass of composition B1 or of compositions B1 and C, relative to the total mass of compositions A and B1, or of compositions A, B1 and C, respectively, is in the range of 40% to 60%.

11. The product as claimed in any one of claims 1 to 10, **characterized in that** the chitosan has an average molecular weight M_{w} in the range of 100 to 700 kg/mol.

12. The product as claimed in any one of claims 1 to 11, **characterized in that** the chitosan has a degree of acetylation in the range of 0 to 20%.

13. The product as claimed in any one of claims 1 to 12, **characterized in that** compositions A and B1 are packaged separately from each other in two compartments of a single packaging unit, and in particular in an injection system such as a two-compartment syringe.

14. The product as claimed in any one of claims 1 to 12, **characterized in that** compositions A and B1 are packaged separately from each other in two separate packaging units.

15. A process for the preparation of a glycerophosphate-free injectable composition containing particles of one or more bioactive glass(es) in suspension in an aqueous solution of chitosan, **characterized in that** it comprises or consists exclusively of the following successive steps:
- provide an aqueous solution of chitosan, called composition A,
- a powder of particles of one or more bioactive glass(es), called composition B1,
- place composition B1 in aqueous suspension in a composition C,
- mix composition A and the aqueous suspension of particles of at least one bioactive glass obtained, rapidly after the suspension of composition B1, preferably less than 20 seconds after, and preferentially less than 10 seconds after,
the different compositions A, B1 and C being defined according to claims 1 to 12 and being glycerophosphate-free.

16. An injectable composition corresponding to a suspension of one or more bioactive glass (es) in an aqueous solution of chitosan, obtained by mixing compositions A and B1, by mixing compositions A, B1 and C, or by mixing compositions B1 and C to form an intermediate composition B2 and then B2 and C, of the product as defined in any one of claims 1 to 12.

17. An injectable composition corresponding to a suspension of one or more bioactive glass (es) in an aqueous solution of chitosan, obtainable by a process defined in any one of claims 15 or 16.

18. The injectable composition as claimed in any one of claims 16 or 17, for use in the regeneration of bone tissue after injection into a subject.

19. An injectable composition for use as claimed in claim 18, wherein after injection into a subject, a chitosan hydrogel is formed *in vivo.*

20. A device for packaging a product as claimed in any one of claims 1 to 12, said device comprising at least two independent compartments respectively containing each of said compositions A and B1.
